# FASCICULE DE BREVET EUROPEEN

(11) **EP 1 052 996 B1**
(45) Date de publication et mention de la délivrance du brevet: **28.07.2004**
(21) Numéro de dépôt: 99901702.3
(22) Date de dépôt: 03.02.1999
(51) Int. Cl.: A61K 31/70

(54) **MEDICAMENT POUR LE TRAITEMENT DES DEREGLEMENTS DE L'APOPTOSE CONTENANT DES OLIGOSACCHARIDES**
ZUSAMMENSETZUNG VON OLIGOSACCHARIDEN ZUR REGULIERUNG VON APOPTOSE
MEDICINE FOR TREATING APOPTOSIS DYSFUNCTION CONTAINING OLIGOSACCHARIDES

(30) Priorité: 03.02.1998 FR 9801237
(43) Date de publication de la demande: 22.11.2000
(73) Titulaire: LABORATOIRES GOEMAR S.A., 35400 Saint-Malo (FR)
(72) Inventeur: YVIN, Jean-Claude, F-35400 Saint Malo (FR); CRUZ, Florence, F-35400 Saint Malo (FR); DESCAMPS, Valérie, F-29680 Roscoff (FR); RICHARD, Christophe, F-29400 Plougourvest (FR); THIBAL, Vesna, F-69002 Lyon (FR); ARRIGO, Patrick, F-74930 Pers-Jussy (FR); CLOAREC, Bernard, F-29250 Saint Pol de Léon (FR)
(74) Mandataire: Koch, Gustave
(86) Numéro de dépôt international: PCT/FR1999/000229
(87) Numéro de publication internationale: WO 1999/039718

(56) Documents cités:
- EP-A- 0 552 373
- EP-A- 0 795 560
- WO-A-95/02684

## Description

L'invention a pour objet un médicament pour le traitement des dérèglements de l'apoptose.

On désigne par le terme "apoptose" la mort cellulaire programmée ou suicide cellulaire.

Cette mort correspond à une auto-élimination des cellules suivant un programme défini.

Elle se traduit tout d'abord par des renflements au niveau de la membrane plasmatique, renflements qui sont accompagnés d'un changement structurel de la membrane, puis par une perte de volume de la cellule qui semble se contracter et s'effondrer sur elle-même.

Le noyau se condense et l'ADN est clivé en petits morceaux (Raff, *"Nature",* 356, 397, 1992; Bortner et al., *"Trends in Cell. Biol."* 5, 21, 1995).

In vivo, la cellule en apoptose est reconnue par les macrophages qui vont la phagocyter et l'éliminer en l'absence de tout processus inflammatoire.

In vivo toujours, l'apoptose est largement utilisée par les organismes vivants pour contrôler les populations cellulaires, en particulier les lymphocytes suite à leur activation.

Par ailleurs, l'apoptose joue, au cours du développement des organismes, un rôle primordial dans l'élimination des tissus embryonnaires non nécessaires (queue du lézard, ébauche des organes génitaux d'un sexe ou de l'autre), et dans la sculpture de l'organisme (élimination des palmures interdigitales entre les futurs doigts et autres).

Certains composés présents dans les organismes vivants induisent spécifiquement un phénomène apoptotique. Ainsi, par exemple chez les mammifères, la liaison du ligand Fas au récepteur membranaire Fas, également désigné par APO-1 ou CD95, induit spécifiquement une apoptose; cette apoptose est utilisée par l'organisme vivant pour contrôler les populations de lymphocytes, notamment de lymphocytes T.

Les susdits récepteur et ligand représentent un système physiologique extrêmement intéressant qui est impliqué dans l'élimination spécifique de cellules qui ne sont plus désirées dans l'organisme.

On peut citer en particulier l'élimination cellulaire au cours de la maturation et de l'activation des lymphocytes T. En fait, le système Fas, c'est-à-dire Fas ligand/Fas récepteur, joue un rôle primordial dans l'homéostasie du système immunitaire.

Le récepteur Fas est un membre d'une famille de protéines qui agissent comme récepteurs à la surface des cellules et qui comprennent également les récepteurs TNF (facteur de nécrose tumorale) et NGF (facteur de croissance des nerfs).

Le récepteur Fas est exprimé dans de nombreuses cellules; il s'accumulerait au niveau de l'appareil de Golgi.

Le mécanisme par lequel le système Fas induit la mort cellulaire est inconnu mais fait appel à l'activation des protéases connues sous l'appellation ICE-like ("interleukine-1 béta-converting enzyme" en anglais) ou caspases.

On peut noter que le ligand Fas peut être sécrété par les cellules pour induire leur propre suicide; mais étant donné que ce ligand se retrouve aussi à la surface de cellules activatrices, celles-ci vont de ce fait induire le suicide de cellules cibles par simple contact. Une fois activé, le récepteur Fas interagit avec de nombreuses protéines intracellulaires pour transmettre le signal déclenchant l'apoptose.

In vitro, il existe d'autres moyens pour induire une apoptose, par exemple en inhibant l'activité de certaines kinases, et en particulier la kinase C; dans ce cas, on peut avoir recours à la staurosporine.

Ce produit est très efficace pour induire la mort des cellules par apoptose.

Par ailleurs, le brevet EP 795 560 décrit un oligosaccharide sulfate de keratan comme agent capable d'induire une apoptose.

Il est néanmoins à remarquer que la transduction des signaux induits par la staurosporine est différente de celle faisant intervenir le récepteur Fas.

Toutefois, si les moyens d'activer l'apoptose sont différents, l'exécution du programme de mort induit par ces deux modes d'activation est équivalente et caractérisée par une activation de la cascade des caspases et un dérèglement de la mitochondrie qui relâche des composés (par exemple le cytochrome C) qui vont promouvoir la destruction programmée de la cellule. Ce phénomène est énergie-dépendant mais ne requiert pas la synthèse de nouvelles protéines. En fait, tout est prêt dans une cellule pour assurer sa propre destruction.

In vivo, la régulation du phénomène apoptotique a une importance considérable.

En effet, de nombreuses pathologies sont associées à son dérèglement.

On peut citer, par exemple, deux cas de dérèglement de l'apoptose dans lesquels celle-ci est modulée via le système Pas: il s'agit des maladies auto-immunes dans lesquelles l'apoptose est déficiente et de la destruction des lymphocytes T CD4+ infectés par HIV-1 dans lesquels l'apoptose est trop active.

Dans d'autres cas tels que les dégénérescences neuronales rencontrées par exemple dans la sclérose en plaques, l'apoptose est activée par des voies non encore connues.

Il existe d'autres pathologies dans lesquelles l'apoptose est déficiente; à cet égard, on peut citer l'accumulation de cellules cancéreuses dont l'apoptose semblerait dépendre du système FAS ("*Green*", Science, vol.278, 1246, 1997).

La Société Demanderesse, au vu des constatations rappelées ci-dessus, a eu le mérite de trouver que, dès lors que l'on dispose d'un médicament capable de moduler les dérèglements de l'apoptose tant du point de vue d'une activation dans le cas des pathologies du groupe de celles comprenant les maladies auto-immunes et les pathologies du type cancer que du point de vue de son inhibition dans le cas des pathologies du groupe de celles comprenant le SIDA, il devenait possible de lutter contre ces maladies.

Et elle a eu le mérite non moins grand de trouver que certaines substances oligosacchari-diques et monosaccharidiques comportant éventuellement, sur au moins certains de leurs motifs unitaires, au moins un substituant du groupe comprenant les groupements sulfate, méthyle et acétyle, étaient propres à moduler les dérèglements de l'apoptose.

L'invention a donc pour objet un médicament caractérisé par le fait qu'il comporte, à titre de principe actif, une quantité efficace d'au moins une substance oligosaccharidique propre à moduler les dérèglements de l'apoptose, et comportant éventuellement, sur au moins certains de ses motifs unitaires, au moins un substituant du groupe comprenant les groupements sulfate, méthyle et acétyle, ladite substance étant choisie dans le groupe comprenant
- les oligosaccharides dérivés par voie enzymatique ou chimique des polymères du groupe comprenant les β 1-3 glucans comportant éventuellement des ramifications β 1-6,
- les oligosaccharides dérivés par voie enzymatique ou chimique des carraghénanes, des agars et des porphyranes.

Selon un mode de réalisation avantageux, le médicament conforme à l'invention comporte, à titre de principe actif, une quantité efficace d'au moins un oligosaccharide propre à moduler les dérèglements de l'apoptose et répondant à la formule 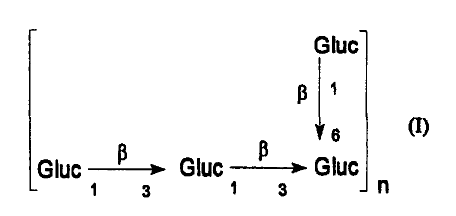 dans laquelle n représente un nombre entier de 1 à 50, de préférence de 5 à 10 et dans laquelle le nombre de branchements varie de 0 à 3 par unité de répétition.

Selon un autre mode de réalisation avantageux, le médicament conforme à l'invention comporte, à titre de principe actif, une quantité efficace d'au moins un disaccharide de répétition propre à moduler les dérèglements de l'apoptose et répondant la formule 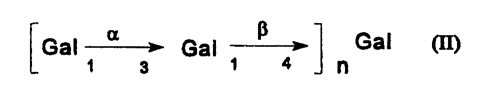 dans laquelle n représente un nombre entier de 1 à 50, de préférence de 1 à 20, au moins certains des disaccharides de répétition de formule (II) pouvant comporter un ou plusieurs groupes sulfate.

Selon un autre mode de réalisation avantageux, le médicament conforme à l'invention comporte, à titre de principe actif, une quantité efficace du produit propre à inhiber au moins partiellement l'apoptose et obtenu par hydrolyse à partir de iota-carraghénate de sodium, ce produit étant constitué par un mélange d'oligo-iota-carraghénanes désigné par I₉, dont la teneur en oses totaux (déterminée selon Tillmans et Philippi) est de 62% et dont le profil de distribution par taille, estimé par électrophorèse sur gel de polyacrylamide selon Zablakis et Perez, est:

| | | |
|---|---|---|
| Iota-néocarratétraose | (DP 2) | 8-12% |
| Iota-néocarrahexaose | (DP 3) | 23-27% |
| Iota-néocarraoctaose | (DP 4) | 18-22% |
| Iota-néocarradécaose | (DP 5) | 13-17% |
| Iota-néocarradodécaose | (DP 6) | 8-12% |
| Oligo-iotacarraghénane Oligo-iotacarraghénanes constitués par 8 | (DP 7) | 3- 7% |
| à 15 disaccharides de répétition | (DP 8-15) | 13-17%. |

Les susdites méthodes sont décrites, pour ce qui est de Zablakis E. & Perez J., dans "*Botanica marina*", 33, 273-276 (1990) et, pour ce qui est de Tillmans J. & Philippi K., dans *"Biochem.* Z.", 215, 30-60 (1930).

Pour préparer le produit I₉, on peut procéder comme suit.

On incube le iotacarraghénane en présence de l'enzyme iotacarraghénase partiellement purifiée à une température de 45 - 50°C, puis on ultrafiltre les produits d'hydrolyse sur une membrane de 10 000 Da. On obtient ainsi le produit I₉.

Plus particulièrement, le polymère de iotacarraghénane est hydrolysé par une iotacarraghénase recombinante surexprimée dans la souche *Escherichia coli.*

La préparation de l'enzyme se fait par dissolution du culot bactérien (correspondant à 1 litre de culture) dans 50 ml de tampon Tris 10 mM pH 7.5, 100 mM NaCl, 5 mM CaCl₂, de façon à avoir au final, 500 U/ml.

Du point de vue pratique, on dissout 100 g de substrat iotacarraghénane dans 20 l d'eau distillée à chaud (80°C) de façon à obtenir à une concentration à 5 g/l puis on ajuste le pH à 7,5 avec du carbonate d'ammonium.

Pour effectuer l'hydrolyse, l'enzyme est ajoutée à 50 U/g de polymère. L'ultrafiltration en continu est engagée après 30 minutes; il s'agit d'une ultrafiltration tangentielle.

Pour cette ultrafiltration tangentielle, on peut utiliser un appareil de marque Pellicon comportant une cassette de 10 000 Da 0.46 m² PTGC de la Société Millipore; cet appareil est réglé sur 2 bars en entrée et 0.5 bar en sortie.

La sortie du filtrat est partiellement fermée pour maintenir le débit de filtration à 1 litre par heure.

Les caractéristiques de l'enceinte réactionnelle sont choisies de façon à permettre un approvisionnement de l'enzyme en substrat jusqu'à épuisement des 20 l de solution et le maintien d'un volume fixe de 2 litres.

On obtient 18 l d'un ultrafiltrat qui est concentré jusqu'à 1 litre par évaporation rotative, puis le concentrat est lyophilisé. Le lyophilisat ainsi obtenu contient le produit I₉.

Les oligocarraghénanes de la fraction I₉ ainsi obtenues ont été soumis à un fractionnement supplémentaire par chromatographie basse pression sur colonne de Biogel P6 puis sur colonne de Sephadex G10.

On obtient ainsi les fractions identifiées plus haut.

Selon un autre mode de réalisation avantageux, le médicament conforme à l'invention comporte, à titre de principe actif, une quantité efficace du produit propre à inhiber au moins partiellement l'apoptose et constitué par la fraction DP 7 du produit I₉.

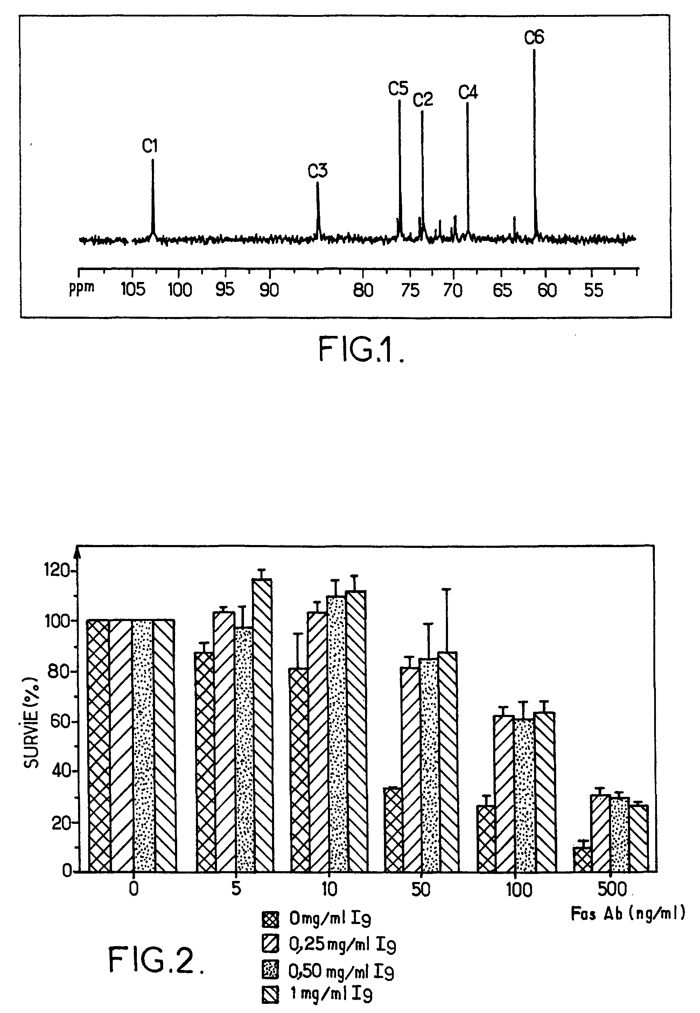

Selon un autre mode de réalisation avantageux, le médicament conforme à l'invention comporte, à titre de principe actif, une quantité efficace du produit propre à activer les dérèglements de l'apoptose, obtenu par extraction aqueuse acide à partir d'une algue brune dénommée *Laminaria digitata*, ce produit étant constitué par un mélange d'oligo β 1-3 glucans désignés par L₁₁ et comportant de 1 à 50, de préférence de 20 à 30 unités saccharidiques, le produit en question présentant le spectre RMN montré à la figure 1.

Il est à noter que le produit L₁₁ peut également être obtenu par extraction aqueuse à partir des algues brunes en général dont *Laminaria digitata* est un représentant.

La préparation du produit L₁₁ peut être effectuée comme suit.

A 300 g d'algues fraîches de type *Laminaria digitata* récoltées au mois d'août sous forme fraîche ou sèche, on ajoute progressivement 1 l d'acide sulfurique 0,3%.

L'opération est réalisée au bain-marie à une température d'environ 70°C pendant 2 heures et 30 minutes sous agitation.

Cette opération est renouvelée deux fois.

L'extrait obtenu est clarifié par filtration sur un filtre de porosité 1,2 µm.

Le liquide résultant de cette filtration est soumis à une ultrafiltration tangentielle sur une membrane de porosité 50000 Daltons.

L'ultrafiltration est réalisée en maintenant une pression de 1 bar.

On obtient ainsi un ultrafiltrat dont le pH est ramené à 5,5 présentant un volume d'environ 0,8 litre. Cet ultrafiltrat est soumis à une dialyse sur une membrane en ester de cellulose de porosité égale à 500 Daltons.

On obtient un dialysat qui est concentré à un volume de 100 ml par évaporation à 80°C à l'aide d'un dispositif de type ROTOVAPOR, puis lyophilisé.

On obtient 7 g d'une poudre couleur crème constituant le produit L₁₁·

Une analyse par chromatographie ionique couplée à l'ampérométrie et utilisant une résine échangeuse d'ions commercialisée par la Société DIONEX, montre que les oligo β 1-3 glucanes constitutifs de la susdite poudre ont en fait 1 à 50, de préférence de 20 à 30 unités saccharidiques.

Les conditions chromatographiques (méthode dite HPLC, c'est-à-dire "Chromatographie liquide sous haute pression") étant les suivantes:

| | |
|---|---|
| Colonne | Carbopac PA1 |
| Débit | 1 ml/min |
| Détection | ampérométrique - électrode en or |
| Injection | 50 µl |
| Gradient d'élution | soude 50 mM / acétate de sodium 500 mM - eau déminéralisée |
| Temps d'analyse | 15 minutes |
| Temps de rétention | ≈ 9 - 10 minutes |

on a obtenu la courbe qui est montrée à la figure 16 et qui identifie le produit L₁₁.

L'examen du spectre RMN du ¹³C du produit L₁₁, réalisé à partir d'une solution à 80 mg/ml dans D₂0 et représenté à la figure 1, montre un squelette de β-D-(1→3)-glucane dont les résonances des différents carbones ont pu être identifiées (elles sont réunies dans le tableau A ci-après) par comparaison avec les valeurs de la littérature [voir Williams et al., 1992 *"Development* of a *water-soluble, sulfated (1→3)-β-D-glucan biological response modifier derived from* Saccharomyces *cerevisiae",* Carbohydr. Res. 235: 247:25].

**TABLEAU I**

| **Déplacements chimiques (ppm) du spectre RMN du** ^{**13**}**C de l'échantillon L**_{**11**} | | |
|---|---|---|
| Squelette de β-D-(1→3)-glucane | C1 | 102,76 |
| | C2 | 73,41 |
| | C3 | 84,94 |
| | C4 | 68,45 |
| | C5 | 75,89 |
| | C6 | 61,06 |
| Résidu D-mannitol | C6 | 63,42 |

Les médicaments conformes à l'invention définis plus haut comportent les adjuvants de formulation classiques correspondant à leur mode d'administration et à la posologie retenues.

L'invention a également pour objet un procédé de préparation d'un médicament pour le traitement des dérèglements de l'apoptose, caractérisé par le fait que l'on fait comporter à une composition galénique au moins l'un des principes actifs identifiés ci-dessus.

Selon un mode de réalisation avantageux, la susdite composition galénique est propre à une administration par voie intraveineuse.

L'invention vise également l'utilisation, en vue de la préparation d'un médicament pour le traitement des dérèglements de l'apoptose, des substances saccharidiques du groupe comprenant les oligosaccharides dérivés par voie enzymatique ou chimique des polymères du groupe comprenant les β 1-3 glucans comportant éventuellement des ramifications β 1-6, et les oligosaccharides dérivés par voie enzymatique ou chimique des galactanes sulfatés, notamment les carraghénanes, les agars et les porphyranes.

Plus particulièrement, elle vise l'utilisation, en vue de la préparation d'un médicament pour le traitement des dérèglements de l'apoptose, des oligosaccharides de formule (I) et de ceux de formule (II).

Plus particulièrement encore, elle vise l'utilisation des produits désignés par I₉ et L₁₁ et des fractions DP 2 et DP 7 de I₉ en vue de la préparation de médicaments pour le traitement des dérèglements de l'apoptose.

L'invention sera encore mieux comprise à l'aide du complément de description qui suit et des exemples qui ne sont nullement limitatifs mais correspondent à des modes de réalisation avantageux.

Dans les expériences qui vont être décrites ci-après, on a travaillé sur des cultures cellulaires dans lesquelles on a déclenché un processus apoptotique en ayant recours au système Fas ou à la staurosporine, puis on a étudié les effets de modulation pouvant être obtenus avec les produits constituant le principe actif des médicaments conformes à l'invention.

Dans le cadre de ces expériences, on a déterminé, d'une part, les quantités appropriées de principe actif pour obtenir l'effet recherché de modulation de l'apoptose et, d'autre part, le ou les moments auxquels il convient d'administrer, pour obtenir l'effet de modulation recherché, le principe actif ou le médicament le comportant.

### EXEMPLE 1

On a travaillé sur une culture de fibroblastes murins génétiquement modifiés pour exprimer constitutivement le récepteur humain Fas; le principe actif testé était le produit désigné par I₉.

Dans une expérience préalable, on a montré que les fibroblastes murins sont détruits par apoptose lorsqu'ils sont mis en présence soit du ligand Fas soit d'un anticorps agoniste reconnaissant le récepteur Fas et désigné dans ce qui suit par FasAb.

Dans une autre expérience préalable, on a déterminé que le produit I₉ n'altérait pas la croissance cellulaire, qu'il n'était pas toxique vis-à-vis des fibroblastes murins aux concentrations utilisées et qu'il pouvait donc être ajouté à un milieu de culture cellulaire sans créer de problèmes.

Le milieu utilisé pour la culture des fibroblastes murins est celui commercialisé par la Société Life Technologies sous l'appellation "Dulbecco's Modified Eagle Medium"; ce milieu est décrit dans *"Virology"* 8, 396 (1959) par Dulbecco et al.

On a ajouté à ce milieu 5% en volume de sérum de veau foetal.

Ce milieu était ensuite inoculé avec des fibroblastes murins en présence d'une quantité suffisante d'antibiotiques pour éliminer les possibilités de contamination; la concentration du milieu de culture en fibroblastes a été de 10⁵ cellules par ml de milieu.

La culture a été effectuée à l'intérieur d'un incubateur dans lequel la température a été maintenue à 37°C; l'atmosphère remplissant l'incubateur contenait 5% de CO₂ .

Après une incubation de 24 heures, on a ajouté soit le Fas ligand, soit le FasAb directement dans le milieu.

La quantité de FasAb ajoutée a été de 50 µg par ml de milieu de culture.

Dans ces conditions, environ 70% des cellules de la culture sont détruites par apoptose après environ 24 heures d'incubation.

Cette destruction est mise en évidence par coloration au cristal violet des cellules survivantes.

On a procédé ensuite à un certain nombre d'essais destinés à mettre en évidence l'action du principe actif.

Dans ces essais, on a fait varier, d'une part, la concentration à laquelle le principe actif est mis en oeuvre dans le milieu de culture et, d'autre part, le moment auquel le principe actif est ajouté à ce milieu de façon à déterminer les concentrations optimales en principe actif ainsi que le ou les moments les plus opportuns d'introduction du principe actif par rapport à l'addition de FasAb.

On a fait varier les concentrations en principe actif de 0,001 à 2 mg par ml.

On a successivement étudié l'effet obtenu en ajoutant d'abord le principe actif avant, puis en même temps et enfin après FasAb.

Dans un premier essai, on a ajouté le principe actif 24 heures avant FasAb.

Dans cadre de cet essai, on a noté l'effet obtenu en utilisant successivement 0, puis 5, puis 10, puis 50, puis 100 et enfin 500 ng de FasAb par ml de culture et, dans chaque cas, des concentrations en principe actif successivement égales à 0,25, puis 0,5 et enfin 1 mg par ml de milieu de culture, étant entendu que l'on a également noté l'effet obtenu en l'absence de principe actif, c'est-à-dire pour une concentration de 0%.

Après 24 heures d'incubation, on procède à l'analyse de survie.

Le résultat de cette analyse est matérialisé par l'histogramme de la figure 2.

Sur l'axe des abscisses de cet histogramme, figure la concentration du milieu en FasAb exprimée en ng/ml et, sur l'axe des ordonnées, le taux de survie exprimé en %.

Pour chacune des concentrations en FasAb, on a matérialisé le taux de survie pour chacune des quatre concentrations en principe actif, soit 0 mg/ml, 0,25 mg/ml, 0,5 mg/ml et 1 mg/ml, par quatre rectangles parallèles à l'axe des ordonnées, l'écart-type étant matérialisé chaque fois par un segment surmontant le rectangle correspondant parallèlement à l'axe des ordonnées.

Le rectangle correspondant à 0 mg/ml de principe actif est chaque fois celui situé le plus à gauche, celui correspondant à 0,25 mg/ml de principe actif est situé sur sa droite, celui correspondant à 0,5 mg/ml est situé à la droite du précédent et ainsi de suite.

Les quatre rectangles sont chaque fois identifiés par des hachures, pointillés ou dégradés particuliers.

L'examen des résultats ainsi réunis sur l'histogramme de la figure 2 montre qu'en l'absence de principe actif, le taux de survie diminue avec l'augmentation de la concentration en FasAb et que ce taux de survie est sensiblement amélioré par l'addition du principe actif.

Dans un deuxième essai, on a ajouté au milieu de culture en même temps le FasAb et le principe actif.

Dans ce deuxième essai, on a noté l'effet obtenu en utilisant successivement les mêmes concentrations en FasAb et en principe actif que dans le premier essai.

Après 24 heures d'incubation, on procède à l'analyse de survie.

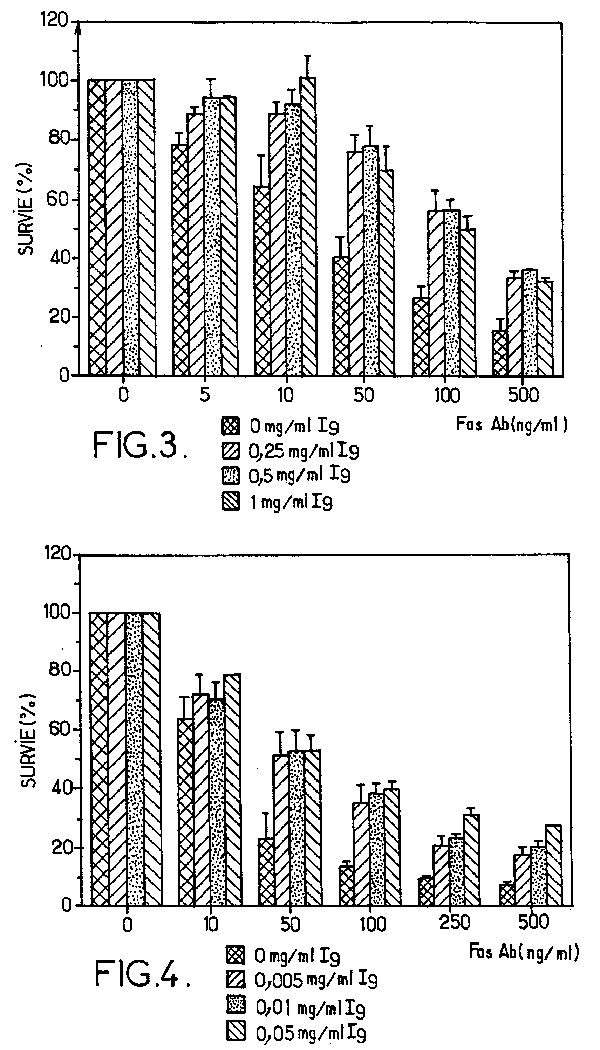

Les résultats enregistrés sont réunis sur l'histogramme de la figure 3 qui est constitué suivant les mêmes principes que ceux exposés à propos de celui de la figure 2.

L'examen de ces résultats montre que le taux de survie évolue d'une manière analogue à celle notée pour le premier essai.

Dans une troisième série d'essais, on a ajouté le principe actif après FasAb, à savoir successivement:
tout d'abord, 1 heure après le FasAb,
ensuite, 3 heures après le FasAb et,
enfin, 6 heures après le FasAb,
en faisant toujours varier la concentration en FasAb de 0 à 500 ng par ml de culture.

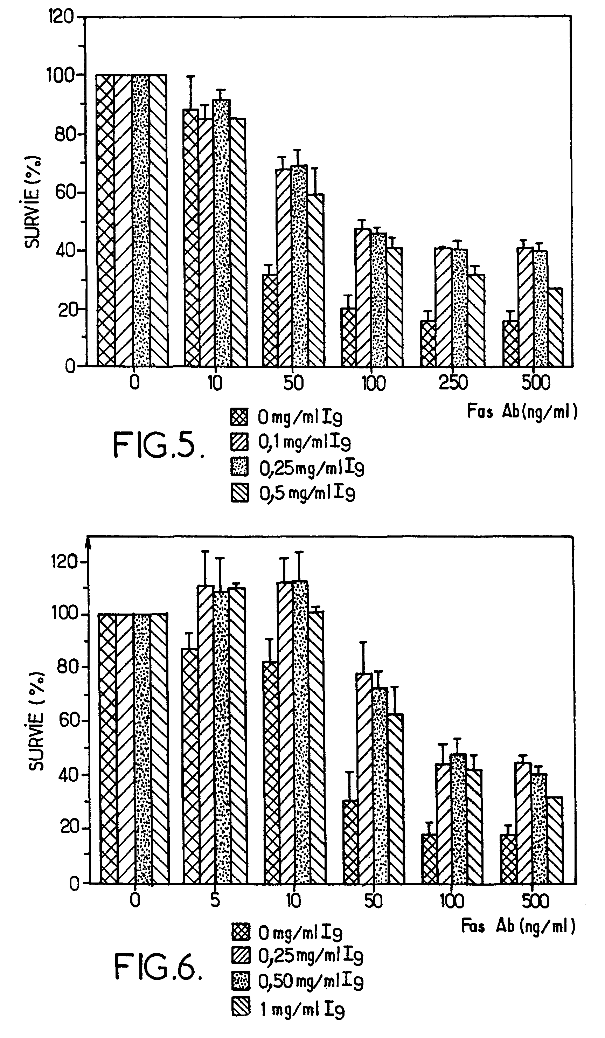

Dans le cas de l'addition de principe actif effectuée 1 heure après le FasAb,
- on a réuni sur l'histogramme de la figure 4 les résultats notés pour des concentrations en I₉ de 0 mg/ml, de 0,005 mg/ml, de 0,01 mg/ml et enfin de 0,05 mg/ml,
- on a réuni sur l'histogramme de la figure 5 les résultats notés pour des concentrations en I₉ de 0 mg/ml, de 0,1 mg/ml, de 0,25 mg/ml et enfin de 0,5 mg/ml, et
- on a réuni sur l'histogramme de la figure 6 les résultats notés pour des concentrations en I₉ de 0 mg/ml, de 0,25 mg/ml, de 0,5 mg/ml et enfin de 1 mg/ml.

Les histogrammes des figures 4 à 6 sont constitués suivant les mêmes principes que ceux exposés à propos de celui de la figure 2.

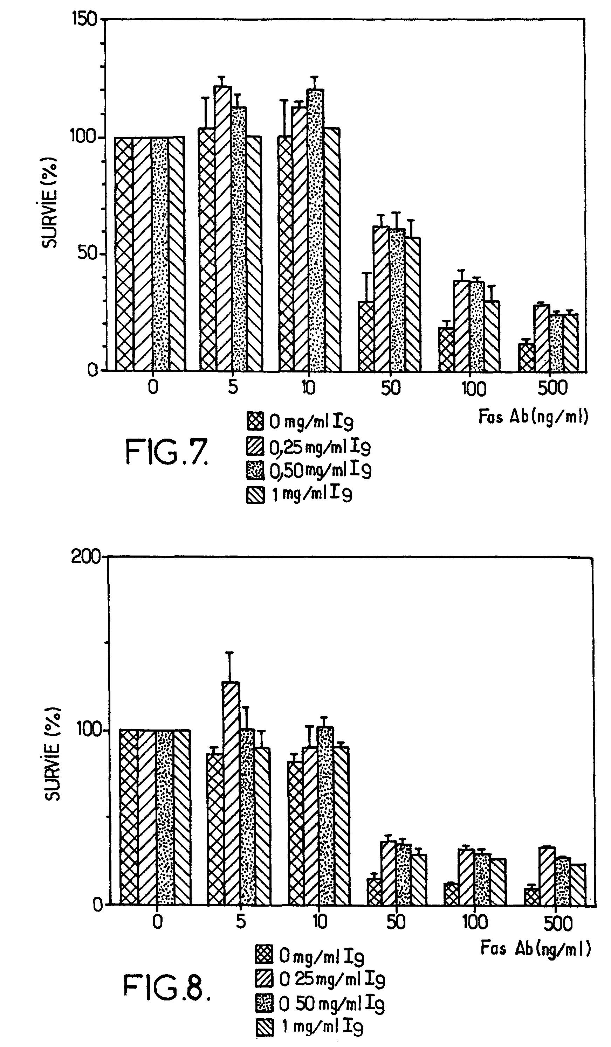

Dans le cas de l'addition de principe actif effectuée 3 heures après l'addition de FasAb, on a réuni sur l'histogramme de la figure 7 les résultats notés pour des concentrations en I₉ de 0 mg/ml, de 0,25 mg/ml, de 0,5 mg/ml et de 1 mg/ml.

Dans le cas de l'addition de principe actif effectuée 6 heures après l'addition de FasAb, on a réuni sur l'histogramme de la figure 8 les résultats notés pour des concentrations en I₉ de 0 mg/ml, de 0,25 mg/ml, de 0,5 mg/ml et de 1 mg/ml.

Les histogrammes des figures 7 et 8 sont constitués suivant les mêmes principes que ceux exposés à propos de celui de la figure 2.

L'examen de l'ensemble des résultats réunis sur les histogrammes des figures 4 à 8 montre que, dans le cas de l'addition du principe actif après l'addition de FasAb, le taux de survie augmente toujours; cet effet a toutefois tendance à diminuer lorsqu'augmente le temps écoulé entre les additions successives de FasAb et de principe actif; il est par ailleurs sensible à la concentration en principe actif lorsque celle-ci est inférieure à 0,25 mg/ml; on n'obtient pas d'amélioration sensible pour les concentrations supérieures à 0,25 mg/ml.

Dans l'expérience qui vient d'être décrite, l'apoptose était induite par le système FasAb.

On a effectué une autre expérience en induisant l'apoptose par l'inhibiteur de kinase constitué par la staurosporine.

Il est rappelé que la staurosporine induit une mort apoptotique dans le cas de la plupart des cellules à des doses variant de 0,5 à 5 µM.

Dans cette expérience, l'addition de staurosporine et de I₉ était simultanée.

On a utilisé deux doses de I₉, à savoir 0,2 mg/ml et 0,5 mg/ml.

La staurosporine a été mise en oeuvre à raison de 0,5 µM puis de 1 µM et enfin de 1,5 µM.

Le taux de survie des cellules traitées a été déterminé dans chaque cas après 18 heures d'incubation.

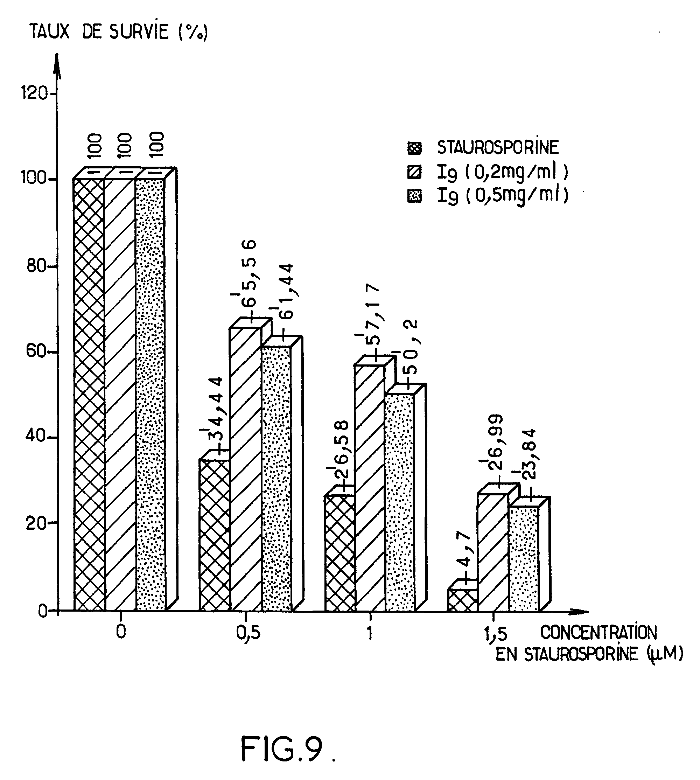

Les résultats enregistrés apparaissent sur l'histogramme représenté à la figure 9 et qui montre le taux de survie exprimé en % en fonction de la concentration en staurosporine et pour les doses de I₉ identifiées plus haut.

Une expérience témoin a été effectuée pour une concentration en staurosporine de 0 µM.

L'examen des résultats apparaissant sur l'histogramme montre que la mise en oeuvre de I₉ atténue l'apoptose induite par la staurosporine.

Il résulte des deux expériences qui viennent d'être décrites que le médicament conforme à l'invention permet d'obtenir une atténuation significative de l'apoptose lorsque celle-ci est induite par différents agents.

### EXEMPLE 2

Dans cet exemple, la culture cellulaire étudiée était une culture de cellules humaines immortalisées, constituées de lymphocytes T (type Jurkat).

Le milieu de culture utilisé est celui commercialisé par la Société Life Technologies sous l'appellation "RPMI 1640 Medium"; ce milieu est décrit par Moore et al. dans la publication "A.M.A." 199, 519 (1967).

On ajoute à ce milieu 10% en volume de sérum de veau foetal et des antibiotiques pour éliminer les possibilités de contamination.

On inocule ce milieu avec une quantité de 10⁶ lymphocytes T par ml de milieu.

La température de l'incubation est de 37°C et l'atmosphère remplissant l'incubateur contient 5% de CO₂.

Après une incubation de 24 heures, on ajoute simultanément le FasAb et le principe actif.

La quantité de FasAb (il s'agit de celui fabriqué par la Société Upstate Biotechnology et commercialisé sous le n° de catalogue 05-201 par la Société Euromedex) ajoutée est de 50 ng/ml de culture.

Le principe actif est constitué successivement par le produit I₉ et le produit L₁₁.

Les quantités mises en oeuvre sont dans chaque cas de 0,5 mg/ml.

L'analyse de survie est effectuée 18 heures après le début de l'expérience.

Cette analyse de survie consiste à faire passer un volume de culture contenant 10⁴ cellules dans un appareil du type de ceux qui fonctionnent par cytométrie de flux, en l'occurrence celui commercialisé par la Société Beckton Dickinson sous l'appellation "FAC Scan cytometer".

Cet appareil utilise une sonde qui détecte la présence de phosphatidyl sérine à la surface des cellules; la présence de ce produit montre que les cellules en question sont apoptotiques.

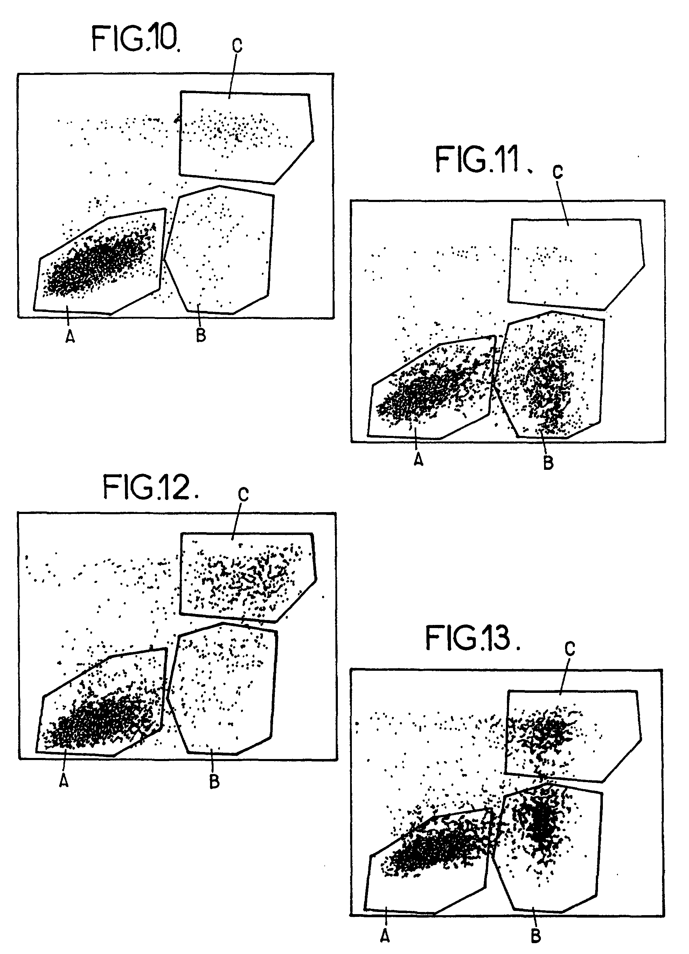

Les résultats de cette analyse apparaissent sur les figures 10 à 13 sur chacune desquelles sont représentés trois polygones, respectivement A, B et C dont les contours sont définis pour être représentatifs de populations cellulaires distinctes; le polygone A entoure un ensemble de cellules vivantes, le polygone B un ensemble de cellules apoptotiques et le polygone C un ensemble de cellules mortes.

Dans le tableau II ci-après, on a réuni les pourcentages de cellules vivantes, de cellules apoptotiques et le pourcentage de cellules mortes dans le cas de chacune des figures 10 à 13 qui vont être commentées ci-après.

**TABLEAU II**

| | | Cellules vivantes Polygone A | Cellules apoptotiques Polygone B | Cellules mortes Polygone C |
|---|---|---|---|---|
| Contrôle | (Fig. 10) | 97 % | 1 % | 1 % |
| FasAb | (Fig.11) | 77 % | 21 % | 1 % |
| FasAb / I₉ | (Fig. 12) | 90 % | 2 % | 6 % |
| FasAb / L₁₁ | (Fig. 13) | 74 % | 13 % | 9 % |

La figure 10 illustre l'analyse de survie effectuée sur un échantillon d'un milieu de culture dans lequel on n'a ajouté ni FasAb, ni principe actif; il s'agit d'un témoin; dans ce cas, on voit qu'il n'y a substantiellement que des cellules vivantes qui se trouvent dans le polygone A (voir ligne 1 du tableau II).

La figure 11 illustre l'analyse de survie effectuée sur un échantillon d'un milieu de culture dans lequel on n'a ajouté que du FasAb; dans ce cas, on voit que le polygone B contient 21% de cellules apoptotiques (voir ligne 2 du tableau II).

La figure 12 illustre l'analyse de survie effectuée sur un échantillon d'un milieu de culture ayant été additionné simultanément de FasAb et de principe actif I₉ (0,5 mg/ml) ; dans ce cas, on voit que le polygone B ne contient pratiquement pas de cellules apoptotiques (2%), le polygone A contenant beaucoup de cellules vivantes et le polygone C une certaine concentration (6%) de cellules mortes (voir ligne 3 du tableau II).

La figure 13 illustre l'analyse de survie effectuée sur un échantillon d'un milieu de culture ayant été additionné simultanément de FasAb et de principe actif L₁₁ (0,5 mg/ml); dans ce cas, on voit que le polygone B contient une quantité importante (13%) de cellules apoptotiques et le polygone C une quantité non négligeable (9%) de cellules mortes (voir liane 4 du tableau II).

Les conclusions susceptibles d'être tirées de l'examen des figures 10 à 13 et de l'analyse du pourcentage des cellules présentes dans les différents polygones font, par conséquent, que le principe actif I₉, dans le cadre de cette expérience, inhibe l'apoptose FasAb (on passe de 21% à 2% de cellules apoptotiques). Toutefois, une légère augmentation du nombre de cellules mortes est notée (ce nombre passe de 1% à 6%). Si on considère le nombre de cellules vivantes, une protection de 13% est observée (on passe de 77% à 90%).

Concernant le principe actif L₁₁, on observe une augmentation du nombre de cellules mortes (on passe de 1% à 9%) par rapport à l'effet induit par FasAb uniquement. Le principe L₁₁, bien que n'induisant pas un effet important sur le nombre de cellules vivantes, agirait donc comme un potentialisateur de la mort cellulaire.

Dans le but d'optimiser l'action de L₁₁, on a réalisé les expériences suivantes.

On administre, en utilisant la même culture que précédemment:
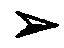 dans une première expérience, 50 ng/ml de FasAb seul (de la provenance Euromedex identifiée plus haut)
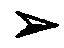 dans une deuxième expérience, la même quantité du même FasAb simultanément à 0,5 mg/ml de produit L₁₁ et
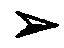 dans une troisième expérience, 0,5 mg/ml du produit L₁₁ puis, 24 heures plus tard, 50 ng/ml du même FasAb.

Les résultats obtenus après 18 heures d'incubation sont comme suit en comparaison avec ce qui est observé sur une culture témoin n'ayant été additionnée ni de FasAb ni de L₁₁, la grandeur prise en considération étant le nombre de cellules vivantes:
- dans le cas d'addition de FasAb seul : le nombre de cellules vivantes diminue de 3,6%,
- dans le cas de l'addition simultanée de FasAb et de L₁₁, le nombre de cellules vivantes diminue de 6,4% et
- dans le cas de l'addition différée de FasAb et de L₁₁, le nombre de cellules vivantes diminue de 13,7%.

Il s'ensuit que L₁₁ est beaucoup plus actif lorsqu'il est administré avant FasAb.

On indique que des résultats analogues ont été obtenus en remplaçant le FasAb identifié ci-dessus par un FasAb d'une autre origine, c'est-à-dire celui fabriqué par la Société Alexis Corporation (San Diego, USA) et commercialisé par la Société Coger SA (Paris).

De l'examen des résultats des expériences qui précèdent, il apparaît qu'un médicament à base du produit L₁₁ permet de potentialiser l'apoptose; on peut donc envisager son utilisation dans le traitement de maladies du type auto-immunes et du cancer.

D'autres expériences ont permis de vérifier les effets produits par l'administration du produit I₉, d'une part, dans des cultures de lymphocytes dans lesquels a été induite une apoptose Fas de faible intensité et, d'autre part, dans le cas des cultures de lymphocytes dans lesquels a été induite une apoptose Fas de forte intensité.

L'apoptose Fas de faible intensité peut être induite en utilisant un FasAb de provenance Euromedex; une telle apoptose peut être de l'ordre de 3 à 10%.

L'apoptose Fas de forte intensité peut être induite en utilisant un FasAb de provenance Alexis Corporation; une telle apoptose peut être de l'ordre de 20 à 50%.

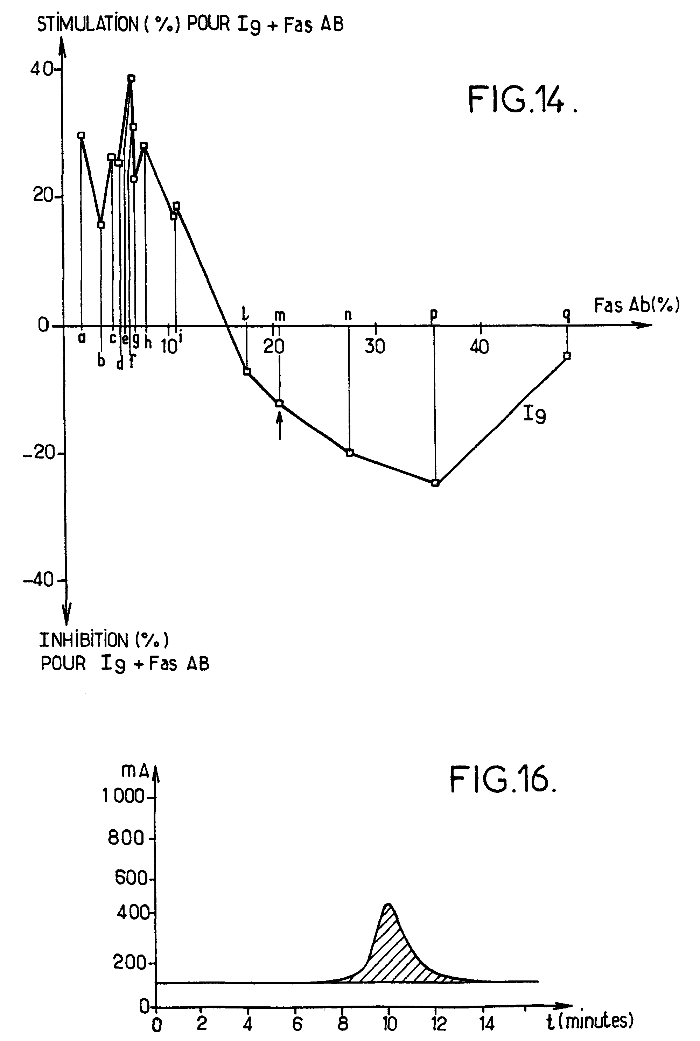

Les expériences réalisées en mettant en oeuvre une dose de 0,2 mg/ml du produit I₉ sont illustrées par la figure 14 qui montre les effets enregistrés, c'est-à-dire soit la stimulation soit l'inhibition de l'apoptose (exprimées en %) en fonction de l'intensité de l'apoptose (en %) induite par FasAb seul (concentrations de 50 à 200 ng/ml).

La figure 14 montre que
- dans le cas d'apoptoses de faible intensité, de l'ordre de 3 à 10%, matérialisées sur l'axe des abscisses par les points a, b, c, d, e, f, g, h, i, l'administration de I₉ provoque une potentialisation de 20 à 40% de l'apoptose par rapport à l'apoptose induite par FasAb seul et
- dans le cas d'apoptoses de forte intensité, de l'ordre de 20 à 50%, matérialisées sur l'axe des abscisses par les points l, m, n, p, q, l'administration de la même quantité de I₉ provoque une inhibition de l'apoptose de 5 à 20% par rapport à l'apoptose produite par FasAb seul.

Sachant qu'il est possible de déterminer chez un sujet donné l'apoptose induite au niveau des lymphocytes, il devient donc possible de moduler celle-ci selon l'intensité constatée de ladite apoptose dans le sens de la potentialisation ou dans le sens de l'inhibition en administrant à ce sujet un médicament à base de I₉.

Ce médicament pourra, par conséquent, permettre de corriger l'apoptose dans le sens de la potentialisation lorsque le sujet est atteint d'une affection du type cancer ou maladie auto-immune correspondant à une apoptose faible et dans le sens d'une inhibition lorsque le sujet est atteint d'une affection du type syndrome immuno-déficient correspondant à une apoptose forte.

Ceci étant, on précise que les lymphocytes T type Jurkat présentes dans les cultures soumises aux expériences qui précèdent, sont des cellules dont le gène suppresseur de tumeur p53 ou protéine p53 (Bing An et al., 1998, "Cell Death and Differentiation" 5, 1062-1075) est muté et, de ce fait, inactif.

Les effets observés avec les produits utilisés conformément à l'invention, notamment avec I₉ et L₁₁, sont donc probablement p53 indépendants; autrement dit, les produits I₉ et L₁₁ sont capables de potentialiser la stimulation ou l'inhibition de l'apoptose de par leur seule présence et indépendamment de la présence ou de l'absence du gene p53 actif.

Or, dans la plupart des cancers humains (voir Hollstein et al., 1994, "Nucl. Acid Res.", 22, 3551), la protéine p53 est mutée, donc inactive; il s'ensuit que la mise en oeuvre d'un médicament à base d'un des produits conformes à l'invention et en particulier des produits I₉ et L₁₁ permet de remédier aux dysfonctionnements de la protéine p53 et de traiter les affections du type cancer et les maladies auto-immunes.

L'invention permet, par conséquent, de traiter les affections en question par une voie fondamentalement différente et plus simple que la thérapie génique qui repose sur le principe visant notamment à réintroduire un gène p53 normal dans le génome des cellules mutées d'un sujet malade.

On sait que la plupart des cellules cancéreuses ont perdu leur sensibilité à l'apoptose FasAb et que la plupart des agents anticancéreux actuellement utilisés agissent en activant le gène p53 qui, lui, agit positivement sur le sytème FasAb. Or, ces systèmes ne fonctionnent plus si p53 est muté (Müller et al. 1998, J. Exp. Med. 188, 2033-2043).

Selon les observations récentes, le système FasAb est capital pour la destruction des cellules cancéreuses, soit par le système immunitaire, soit par l'action des drogues anticancéreuses; il s'ensuit que le fait que les produits utilisés conformément à l'invention et notamment I₉ et L₁₁ modulent l'apoptose desdites cellules cancéreuses de manière indépendante de p53, revêt une importance considérable et permet d'envisager la mise au point d'une nouvelle génération de médicaments notamment anticancéreux.

### EXEMPLE 3

Le produit I₉ est composé, comme montré plus haut, d'un mélange d'oligo-iota-carraghénanes.

On a testé l'effet induit par différents constituants de ce mélange, en particulier des fractions DP 2, DP 3, DP 4, DP 5 et DP 7.

On a également testé le polymère constituant la matière première de I₉, ainsi que le produit dénommé KIK (hexasaccharide de type kappa-iota-kappa).

Pour ces expériences, on a procédé comme indiqué à l'exemple 1 en introduisant simultanément, d'une part, 0,2 mg/ml de chacune des susdites fractions de I₉ et, d'autre part, 100 ng/ml de FasAb d'origine Euromedex.

Après 24 heures d'incubation, on a fait les constatations résumées ci-après.

Les fractions DP 2, DP 3, DP 4 et DP 5 sont inactives et ne stimulent pas l'apoptose induite par une faible dose (100 ng/ml) de FasAb de provenance Euromedex qui induit par elle-même 6% de mort cellulaire après 18 heures d'incubation.

Par contre, un effet stimulateur très fort est obtenu pour la fraction DP 7 (50%) et par le produit I₉ (70%), ce qui montre qu'un des composants actifs du mélange I₉ peut être constitué par la fraction DP 7; par ailleurs, le produit KIK n'a pas d'activité.

On a enfin trouvé que le polymère constituant la matière première pour la préparation de I₉ avant clivage par l'enzyme iotase est, lui aussi, actif et stimule une apoptose Fas de faible intensité. Par contre, l'enzyme iotase elle-même, mise en oeuvre à raison de 50 unités, n'a pas d'activité lorsqu'elle est incubée avec les cellules.

Il s'ensuit que le principe actif I₉, qui n'a pas d'activité apoptotique intrinsèque, induit une très forte stimulation dans le cas d'une apoptose FasAb de faible intensité.

### EXEMPLE 4

Il a été procédé à une expérience complémentaire confirmant les résultats traduits par la figure 14.

Dans le cas des phénomènes apoptotiques induits par Fas, la première des caspases de la cascade de caspases activées est la caspase 8.

Il a été procédé à la mesure de l'activité de cette capsase 8, qui est une enzyme protéolytique, après lyse des cellules Jurkat traitées ou non avec FasAb seul et avec FasAb en même temps qu'avec I₉.

Pour ce faire, on a mesuré
- l'activité de la caspase 8 dans une première fraction d'une culture de cellules Jurkat en l'absence de FasAb et de I₉; le pourcentage de cellules vivantes dans la culture est de 93%;
- l'activité de la caspase 8 dans une deuxième fraction de la même culture, 18 heures après incubation avec 500 ng/ml de FasAb de provenance Euromedex (qui, comme indiqué plus haut, induit une faible apoptose, de l'ordre de 13%); le pourcentage de cellules vivantes dans la culture est de 80%;
- l'activité de la caspase 8 dans une troisième fraction de la même culture, 18 heures après incubation avec 500 ng/ml de FasAb de provenance Euromedex et de 0,2 mg/ml de I₉; le pourcentage de cellules vivantes dans la culture est de 50%;
- l'activité de la caspase 8 dans une quatrième fraction de la même culture, 18 heures après incubation avec 25 ng/ml de FasAb de provenance Alexis (qui, comme indiqué plus haut, induit une apoptose intense, de l'ordre de 40%) ; le pourcentage de cellules vivantes dans la culture est de 53%;
- l'activité de la caspase 8 dans une cinquième fraction de la même culture, 18 heures après incubation avec 25 ng/ml de FasAb de provenance Alexis et de 0,2 mg/ml de I₉; le pourcentage de cellules vivantes dans la culture est de 59%;

La mesure de l'activité caspase a été effectuée chaque fois à l'aide du kit commercialisé par la Société Ozyme sous la dénomination "Apo Alert Flice FLuor" No. K2028-2.

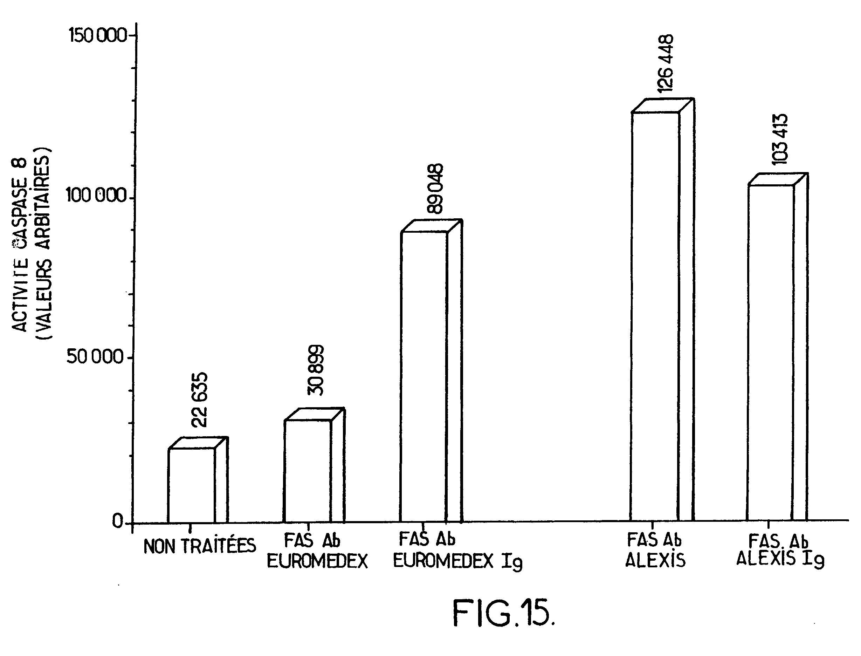

Les résultats de ces mesures sont réunis dans l'histogramme de la figure 15.

L'examen de cet histogramme montre que
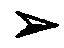 l'activité caspase 8 est faiblement stimulée (facteur voisin de 1,37) par FasAb Euromedex seul, l'administration simultanée de I₉ induisant une stimulation forte (facteur voisin de 3,93);
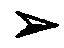 l'activité caspase 8 est fortement stimulée (facteur voisin de 4,96) par FasAb Alexis seul, cette forte stimulation étant diminuée (facteur voisin de 4,05) lorsque I₉ est introduit simultanément à FasAb Alexis.

Il existe, par conséquent, une corrélation nette entre l'apoptose et l'activité caspase 8.

Le produit I₉ représente donc un produit capable de moduler l'activité de la caspase 8.

Le fait que l'on montre que cette enzyme est essentielle dans la voie biochimique de l'apoptose induite par Fas (Juo et al., Curr. Biol. 8, 1001-1008, 1998), permet d'envisager de nouveaux médicaments à base d'oligosaccharides permettant de cibler les enzymes du type caspase.

Ces produits représentent donc une alternative à une thérapie basée sur des peptides portant la séquence reconnue par la caspase 8 ("substrats suicide" de caspase 8).

## Revendications

1. Médicament **caractérisé par le fait qu'**il comporte, à titre de principe actif, une quantité efficace d'au moins une substance oligosaccharidique propre à moduler les dérèglements de l'apoptose et comportant éventuellement, sur au moins certains de ses motifs unitaires, au moins un substituant du groupe comprenant les groupements sulfate, méthyle et acétyle, la substance étant choisie dans le groupe comprenant
- les oligosaccharides dérivés par voie enzymatique ou chimique des polymères du groupe comprenant les β 1-3 glucans comportant éventuellement des ramifications β 1-6, et
- les oligosaccharides dérivés par voie enzymatique ou chimique des des carraghénanes, des agars et des porphyranes.

2. Médicament **caractérisé par le fait qu'**il comporte, à titre de principe actif, une quantité efficace d'au moins un oligosaccharide propre à moduler les dérèglements de l'apoptose et répondant à la formule 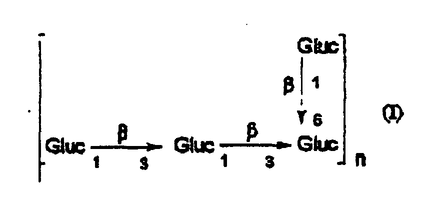 dans laquelle n
représente un nombre entier de 1 à 50, de préférence de 5 à 10 et dans laquelle le nombre de branchements varie de 0 à 3 par unité de répétition.

3. Médicament **caractérisé par le fait qu'**il comporte, à titre de principe actif, une quantité efficace d'au moins un disaccharide de répétition propre à moduler les dérèglements de l'apoptose et répondant à la formule 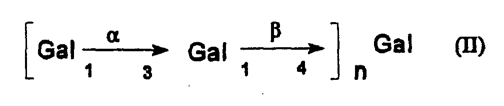 dans laquelle n représente un nombre entier de 1 à 50, de préférence de 1 à 20, au moins certains des disaccharides de répétition de formule (II) pouvant comporter un ou plusieurs groupes sulfate.

4. Médicament **caractérisé par le fait qu'**il comporte, à titre de principe actif, une quantité efficace du produit propre à inhiber au moins partiellement l'apoptose et obtenu par hydrolyse à partir de iota-carraghénate de sodium, ce produit étant constitué par un mélange d'oligo-iota-carraghénanes désigné par I₉, dont la teneur en oses totaux (déterminée selon Tillmans et Philippi) est de 62% et dont le profil de distribution par taille, estimé par électrophorèse sur gel de polyacrylamide selon Zablakis et Perez, est:
| | | |
|---|---|---|
| Iota-néocarratétraose | (DP 2) | 8-12% |
| Iota-néocarrahexaose | (DP 3) | 23-27% |
| Iota-néocarraoctaose | (DP 4) | 18-22% |
| Iota-néocarradécaose | (DP 5) | 13-17% |
| Iota-néocarradodécaose | (DP 6) | 8-12% |
| Oligo-iotacarraghénane Oligo-iotacarraghénanes constitués par 8 | (DP 7) | 3- 7% |
| à 15 disaccharides de répétition | (DP 8-15) | 13-17%. |

5. Médicament **caractérisé par le fait qu'**il comporte, à titre de principe actif, une quantité efficace du produit propre à activer les dérèglements de l'apoptose, obtenu par extraction aqueuse acide à partir des algues brunes et plus particulièrement d'une algue brune dénommée *Laminaria digitata*, ce produit étant constitué par un mélange d'oligo β 1-3 glucans désignés par L₁₁ et comportant de 1 à 50, de préférence de 20 à 30 unités saccharidiques, le produit en question présentant le spectre RMN montré à la figure 1.

6. Médicament **caractérisé par le fait qu'**il comporte, à titre de principe actif, une quantité efficace du produit propre à activer les dérèglements de l'apoptose et constitué par la fraction DP 7 du produit I₉ selon Ia revendication 4.

7. Procédé de préparation d'un médicament pour le traitement des dérèglements de l'apoptose, **caractérisé par le fait que** l'on fait comporter à une composition galénique au moins l'un des principes actifs des médicaments selon au moins l'une des revendications 1 à 6.

8. Utilisation, en vue de la préparation d'un médicament pour le traitement des dérèglements de l'apoptose, d'au moins l'une des substances oligo-saccharidiques comportant éventuellement, sur au moins certains de leurs motifs unitaires, au moins un substituant du groupe comprenant les groupements sulfate, méthyle et acétyle, lesdites substances qui sont propres à moduler les dérèglements de l'apoptose étant choisies dans le groupe comprenant
- les oligosaccharides dérivés par voie enzymatique ou chimique des polymères du groupe comprenant les β 1-3 glucans comportant éventuellement des ramifications β 1-6, et
- les oligosaccharides dérivés par voie enzymatique ou chimique des carraghénanes, des agars et des porphyranes,

9. Utilisation, en vue de la préparation d'un médicament pour le traitement des dérèglements de l'apoptose, des oligosaccharides de formule (I) et de ceux de formule (II).

10. Utilisation des produits désignés par I₉ et L₁₁ et du produit constituant la fraction DP 7 du produit I₉ selon la revendication 4 en vue de la préparation de médicaments pour le traitement des dérèglements de l'apoptose.

## Claims

1. Medicine, **characterized in that** it comprises, as an active principle, an effective amount of at least one oligosaccharide substance which is capable of modifying apoptosis dysfunctions and which optionally comprises, on at least some of its individual units, at least one substituent of the group comprising sulfate, methyl and acetyl groups, said substance being chosen from the group comprising
- the oligosaccharides which are derived, by enzymatic or chemical process, from the polymers of the group comprising (1→3)-β-glucans which optionally comprise (1→6)-β-branching, and
- the oligosaccharides which are derived, by enzymatic or chemical process, from sulfated galactans, in particular carrageenans, agars and porphyrans.

2. Medicine, **characterized in that** it comprises, as an active principle, an effective amount of at least one oligosaccharide which is capable of modifying apoptosis dysfunctions and which satisfies the formula 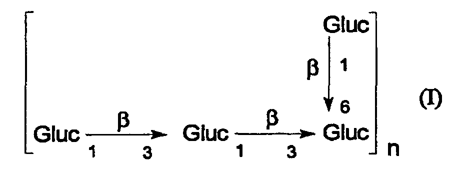 in which n represents an integer from 1 to 50, preferably from 5 to 10, and in which the number of branches varies from 0 to 3 per repeat unit.

3. Medicine, **characterized in that** it comprises, as an active principle, an effective amount of at least one repeat disaccharide which is capable of modifying apoptosis dysfunctions and which satisfies the formula 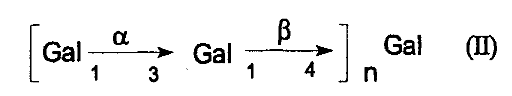 in which n represents an integer from 1 to 50, preferably from 1 to 20, at least some of the repeat disaccharides of formula (II) possibly comprising one or more sulfate groups.

4. Medicine, **characterized in that** it comprises, as an active principle, an effective amount of the product which is capable of at least partially inhibiting apoptosis and which is obtained by hydrolysis from sodium iota-carrageenate, this product consisting of a mixture of oligo-iota-carrageenans which is referred to as I₉, which has a total saccharide content (determined according to Tillmans and Philippi) of 62%, and which has a distribution profile by size, which is estimated by electrophoresis on polyacrylamides gel according to Zablakis and Perez, of
| | | |
|---|---|---|
| iota-neocarratetraose | (DP 2) | 8-12% |
| iota-neocarrahexaose | (DP 3) | 23-27% |
| iota-neocarraoctaose | (DP 4) | 18-22% |
| iota-neocarradecaose | (DP 5) | 13-17% |
| iota-neocarradodecaose | (DP 6) | 8-12% |
| oligo-iota-carrageenan | (DP 7) | 3-7% |
| oligo-iota-carrageenans | | |
| consisting of 8 to 15 repeat disaccharides | (DP 8-15) | 13-17%. |

5. Medicine, **characterized in that** it comprises as an active principle, an effective amount of the product which is capable of activating apoptosis dysfunction, and which is obtained by acidic aqueous extraction from brown algae and more particularly from a brown alga named *Laminaria digitata*, this product consisting of a mixture of oligo-(1→3)-β-glucans which are referred to as L₁₁ and comprise from 1 to 50, preferably from 20 to 30, saccharide units, the product in question having the NMR spectrum shown in Figure 1.

6. Medicine, **characterized in that** it comprises, as an active principle, an effective amount of the product which is capable of activating apoptosis dysfunctions and which consists of fraction DP 7 of the product I₉.

7. Method for preparing a medicine for treating apoptosis dysfunctions **characterized in that** a pharmaceutical composition comprises at least one of the active principles of the medicine according to at least one of Claims 1 to 6.

8. Use, in order to prepare a medicine for treating apoptosis dysfunctions of at least one of the oligosaccharide substances which optionally comprise, on at least some of their individual units, at least one substituent of the group comprising sulfate, methyl and acetyl groups, said substances which are capable of modifying apoptosis dysfunctions being chosen from the group comprising
- the oligosaccharides which are derived, by enzymatic or chemical process, from the polymers of the group comprising (1→3)-β-glucans which optionally comprise (1→6)-β-branching, and
- the oligosaccharides which are derived, by enzymatic or chemical process, from sulfated galactans, in particular carrageenans, agars and porphyrans.

9. Use, in order to prepare a medicine for treating apoptosis dysfunctions of the oligosaccharides of formula (I) and of those of formula (II).

10. Use of the products which are referred to as I₉ and L₁₁ and of the product constituting fraction DP 7 of the product I₉ with a view to preparing medicines for treating apoptosis dysfunctions.

## Patentansprüche

1. Medikament, **dadurch gekennzeichnet, dass** es als Wirkstoff eine wirksame Menge von mindestens einer oligosaccharidischen Substanz, die dazu geeignet ist, Unregelmäßigkeiten der Apoptose zu modulieren, aufweist und gegebenenfalls, auf mindestens einigen seiner Einheitsmotive, mindestens einen Substituenten der Gruppe, umfassend die Gruppierungen Sulfat, Methyl und Acetyl, aufweist, wobei die Substanz ausgewählt ist aus der Gruppe, umfassend
- Oligosaccharide, auf enzymatischem oder chemischem Weg abgeleitet von Polymeren der Gruppe, umfassend β 1-3 Glukane, gegebenenfalls enthaltend β 1-6 Verzweigungen, und
- Oligosaccharide, auf enzymatischem oder chemischem Weg abgeleitet von Carrageenanen, Agaren und Porphyranen.

2. Medikament, **dadurch gekennzeichnet, dass** es als Wirkstoff eine wirksame Menge von mindestens einem Oligosaccharid aufweist, das dazu geeignet ist, Unregelmäßigkeiten der Apoptose zu modulieren und der Formel 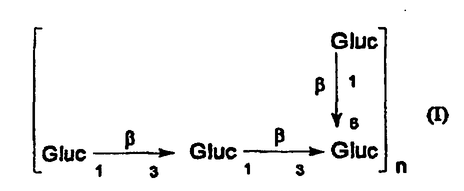 entspricht, in welcher n eine ganze Zahl von 1 bis 50 darstellt, vorzugsweise von 5 bis 10, und in welcher die Zahl der Abzweigungen von 0 bis 3 pro Repetitionseinheit variiert.

3. Medikament, **dadurch gekennzeichnet, dass** es als Wirkstoff eine wirksame Menge von mindestens einem Repetitions-Disaccharid, das dazu geeignet ist, Unregelmäßigkeiten der Apoptose zu modulieren, und der Formel 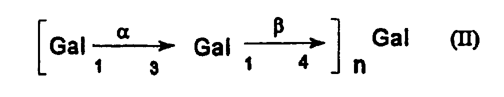 entspricht, in welcher n eine ganze Zahl von 1 bis 50 darstellt, vorzugsweise von 1 bis 20, wobei mindestens einige der Repetier-Disaccharide der Formel (II) eine oder mehrere Sulfatgruppe(n) aufweisen können.

4. Medikament, **dadurch gekennzeichnet, dass** es als Wirkstoff eine wirksame Menge des Produkts aufweist, das dazu geeignet ist, die Apoptose mindestens teilweise zu inhibieren, und das erhalten wird durch Hydrolyse von Nattium Jota-Catrageenat ("iota-carraghénate de sodium"), wobei das Produkt durch ein Gemisch aus Oligo-Jota-Carrageenanen, bezeichnet als I₉, gebildet *wird,* deren Gesamtgehalt an Monosacchariden (bestimmt nach Tillmans und Philippi) 62% beträgt, und deren Größenverteilungsprofil, bestimmt mittels Polyacrylamid-Gelelektrophorese nach Zablakis und Perez, ist:
| | | |
|---|---|---|
| Jota-Neocarratetraose | (DP 2) | 8-12% |
| Jota-Neocarrahexaose | (DP 3) | 23-27% |
| Jota-Neocarraoctaose | (DP 4) | 18-22% |
| Jota-Neocarradecaose | (DP 5) | 13-17% |
| Jota-Neocartadodecaose | (DP 6) | 8-12% |
| Oligo-Jota-Carrageenan | (DP 7) | 3- 7% |
| Oligo Jota-Carrageenanen, gebildet aus 8 bis 15 Repetier-Disacchariden | (DP 8-15) | 13-17%. |

5. Medikament, **dadurch gekennzeichnet, dass** es als Wirkstoff eine wirksame Menge des Produkts aufweist, das dazu geeignet ist, Unregelmäßigkeiten der Apoptose zu aktivieren, und das erhalten wird durch wässerig-sauere Extraktion aus Braunalgen, insbesondere aus einer Braunalge, die als *Laminaria digitata* bezeichnet wird, wobei das Produkt durch ein Gemisch aus Oligo β 1-3 Glukanen, bezeichnet als L₁₁, gebildet wird und von 1 bis 50, vorzugsweise von 20 bis 30 Saccharideinheiten aufweist, wobei das besagte Produkt das NMR-Spektrum präsentiert, das in der Figur 1 gezeigt ist.

6. Medikament, **dadurch gekennzeichnet, dass** es als Wirkstoff eine wirksame Menge des Produkts enthält, das dazu geeignet ist, Unregelmäßigkeiten der Apoptose zu aktivieren, und aus der Fraktion DP 7 des Produkts I₉ nach Anspruch, 4 gebildet wird.

7. Verfahren zur Herstellung eines Medikaments für die Behandlung von Unregelmäßigkeiten der Apoptose, **dadurch gekennzeichnet, dass** es in einer galenischen Zusammensetzung mindestens einen der Wirkstoffe der Medikamente nach mindestens einem der Ansprüche 1 bis 6 aufweisen muss.

8. Verwendung zur Herstellung eines Medikaments zur Behandlung von Unregelmäßigkeiten der Apoptose von mindestens einer der oligosaccharidischen Substanzen, die gegebenenfalls, auf mindestens einigen ihrer Einheitsmotive, mindestens einen Substituenten der Gruppe, umfassend die Gruppierungen Sulfat, Methyl und Acetyl aufweisen, wobei diese Substanzen geeignet sind, Unregelmäßigkeiten der Apoptose zu modulieren und wobei sie ausgewählt sind aus der Gruppe, umfassend
- Oligosaccharide, auf enzymatischem oder chemischem Weg abgeleitet von Polymeren der Gruppe, umfassend β 1-3 Glukane, gegebenenfalls aufweisend β 1-6 Verzweigungen, und
- Oligosaccharide, auf enzymatischem oder chemischem Weg abgeleitet von Carrageenanen, Agaren und von Porphyranen.

9. Verwendung, zur Herstellung eines Medikaments zur Behandlung von Unregelmäßigkeiten der Apoptose, von Oligosacchariden der Formel (I) und denen der Formel (II).

10. Verwendung von Produkten, bezeichnet als I₉, und L₁₁ und des Produkts, das die Fraktion DP 7 des Produkts I₉ nach Anspruch 4 bildet, zur Herstellung von Medikamenten zur Behandlung von Unregelmäßigkeiten der Apoptose.
